# EUROPEAN PATENT SPECIFICATION

(11) **EP 3 928 096 B1**
(45) Date of publication and mention of the grant of the patent: **02.07.2025**
(21) Application number: 20708394.0
(22) Date of filing: 21.02.2020
(51) Int. Cl.: G01N 33/574

(54) **USE OF BMMF1 REP PROTEIN AS A BIOMARKER FOR PROSTATE CANCER**
VERWENDUNG VON BMMF1-REP-PROTEIN ALS BIOMARKER FÜR PROSTATAKREBS
UTILISATION DE PROTÉINE REP BMMF1 EN TANT QUE BIOMARQUEUR POUR LE CANCER DE LA PROSTATE

(30) Priority: 22.02.2019 EP 19158840
(43) Date of publication of application: 29.12.2021
(73) Proprietor: Deutsches Krebsforschungszentrum Stiftung des öffentlichen Rechts, 69120 Heidelberg (DE)
(72) Inventor: BUND, Timo, 69221 Dossenheim (DE); DE VILLIERS-ZUR HAUSEN, Ethel-Michele, 69483 Waldmichelbach (DE); ZUR HAUSEN, Harald, 69483 Waldmichelbach (DE); ERNST, Claudia, 69436 Schönbrunn (DE); TESSMER, Claudia, 74869 Schwarzach (DE)
(74) Representative: Schüssler, Andrea
(86) International application number: PCT/EP2020/054617
(87) International publication number: WO 2020/169798

(56) References cited:
- WO-A1-2010/028646
- WO-A2-2016/005054
- KIRAN GOLLAPUDI ET AL: "Association between tumor-associated macrophage infiltration, high grade prostate cancer, and biochemical recurrence after radical prostatectomy", AM J CANCER RES, 1 January 2013 (2013-01-01), pages 523 - 529, XP055609108, Retrieved from the Internet <URL:https://www.ncbi.nlm.nih.gov/pmc/articles/PMC3816972/pdf/ajcr0003-0523.pdf> [retrieved on 20190726]
- SEBASTIAN EILEBRECHT ET AL: "Expression and replication of virus-like circular DNA in human cells", SCIENTIFIC REPORTS, vol. 8, no. 1, 12 February 2018 (2018-02-12), pages 1 - 15, XP055575102, DOI: 10.1038/s41598-018-21317-w
- LIPPI GIUSEPPE ET AL: "Meat consumption and cancer risk: a critical review of published meta-analyses", CRITICAL REVIEWS IN ONCOLOGY/HEMATOLOGY, vol. 97, January 2016 (2016-01-01), pages 1 - 14, XP029373173, ISSN: 1040-8428, DOI: 10.1016/J.CRITREVONC.2015.11.008

## Description

The invention generally relates to the use of a DNA-replication-associated (Rep) protein as a biomarker for prostate cancer. More specifically, the present invention refers to the use of Bovine Meat and Milk Factor Group 1 (BMMF1) Rep Protein as a biomarker for prostate cancer, wherein the Rep protein is a MSBI1 genome-encoded Rep protein (MSBI1 Rep) encoded by MSBI1.176 having the amino acid sequence as depicted in SEQ ID NO: 1, a MSBI2 genome-encoded Rep protein (MSBI2 Rep) encoded by MSBI2.176 having the amino acid sequence as depicted in SEQ ID NO: 8, a CMI1 genome-encoded Rep protein (CMI1 Rep) encoded by CMI1.252 comprising the amino acid sequence as depicted in SEQ ID NO: 10, a CMI2 genome-encoded Rep protein (CMI2 Rep) encoded by CMI2.214 comprising the amino acid sequence as depicted in SEQ ID NO: 11 or a CMI3 genome-encoded Rep protein (CMI3 Rep) encoded by CMI3.168 comprising the amino acid sequence as depicted in SEQ ID NO: 12, or a variant of the protein with SEQ ID NO: 1 or SEQ ID NO 8 having a homology of at least 90%, wherein the variant has one or more amino acid deletions, substitutions or additions and is capable of binding an anti-Rep antibody specific for a Rep protein having the amino acid sequence of SEQ ID NO: 1 or SEQ ID NO: 8.

### Background of the Invention

Prostate cancer is the second most common cause of cancer mortality in men in the United States. Over 200,000 new cases are identified each year and over 30,000 will die from this disease this year alone.

Most prostate cancer is initially androgen dependent i.e., prostate cancer cells require androgen for continued proliferation. Androgen deprivation therapy (ADT) through either surgery or medical treatment rapidly leads to apoptosis of androgen-dependent cancer cells. ADT has been the mainstay of treatment for metastatic hormone sensitive prostate cancer (mHSPC) for more than 70 years.

In many cases, however, some cancer cells survive and become androgen independent or unresponsive, leading to recurrence of prostate cancer.

Chemotherapy has been reserved for metastatic castration-resistant prostate cancer (mCRPC), a type of androgen-independent prostate cancer. Taxanes and DNA damaging agents are two major classes of chemotherapeutics used for treating prostate cancer.

Detection of prostate cancer early provides the best opportunity for a cure. Although prostate specific antigen (PSA) is considered as an effective tumor marker, it is not cancer specific. There is considerable overlap in PSA concentrations in men with prostate cancer and men with benign prostatic diseases. Furthermore, PSA levels cannot be used to differentiate men with indolent or organ confined prostate cancer (who would benefit from surgery) from those men with aggressive or non-organ confined prostate cancer (who would not benefit from surgery).

At present, serum PSA measurement, in combination with digital rectal examination (DRE), represents the leading tool used to detect and diagnose prostate cancer. Commercially available PSA assays are commonly performed in regional or local laboratories. These assays play apart in the current strategy for early detection of prostate cancer.

Because advanced disease is incurable, efforts have focused on identifying prostate cancer at an early stage when it is confined to the prostate and therefore more amenable to cure. Unfortunately, prostate cancer can remain asymptomatic until tumor metastasis affects other organs or structures. Screening for prostate cancer is primarily done by the detection of PSA in the blood although the diagnostic value of PSA for prostate cancer is limited, due to its lack of specificity between benign and cancerous conditions. As mentioned above, PSA is not a disease-specific marker, as elevated levels of PSA are detectable in a large percentage of patients with benign prostatic hyperplasia (BPH) and prostatitis (25-86%), as well as in other nonmalignant disorders, which significantly limits the diagnostic specificity of this marker.

Thus, despite screening programs many patients are diagnosed late due to the lack of predictive biomarkers other than PSA. To enhance earlier detection, there is a need for biomarkers that will facilitate early detection and further insights into the pathogenesis of prostate cancer.

In connection with the detection of prostate cancer, WO 2010/028646 A1 refers to the diagnosis of prostate cancer based on the use of villin as a prostate biomarker. It further relates to compositions comprising anti-villin antibodies and the use of these compositions for an improved detection of prostate cancer in a subject.

Gollapudi K. et al. 2013 is mentioning the potential role of TAMs (tumor-associated macrophages) in prostate cancer development, wherein a tissue microarray of prostatectomy specimens was stained for CD68 as a TAM marker.

### Description of the Present Invention

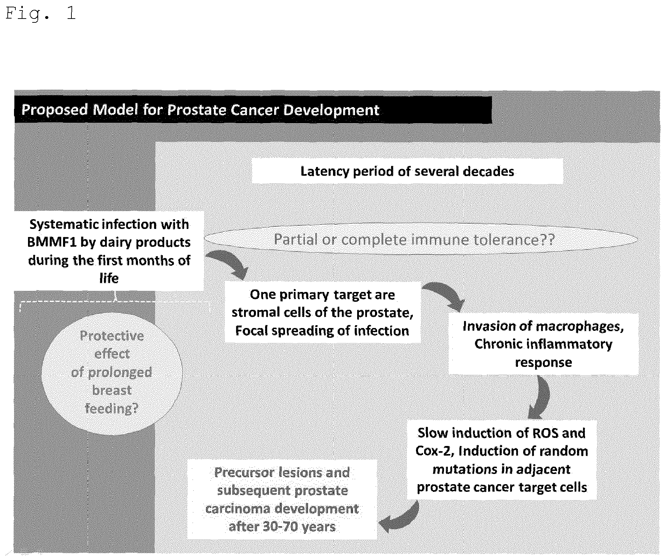

In the present application the inventors have created a model for prostate cancer development that is shown in Fig. 1.

It is generally known that meat consumption may be associated with an enhanced cancer risk (Lippi et al., 2015). The inventors found that the uptake of BMMF (Bovine Meat and Milk Factor) agents within the first months of life either by substitution of breast-feeding during weaning by cow milk products or by the uptake of dairy or beef products, in general, leads to the early infection of newborns with BMMF antigens. Based on the decline of maternal antibodies and the frequently observed weakness of the immune system often coupled with induction of immune tolerances of the newborn during this very early period of life, these agents might either directly escape immune response or a situation of immune tolerance against these agents might be induced. Within the next years or decades - depending on the immune system of the host - more and more BMMF antigens accumulate within the stroma of the prostate tissue. This accumulation may be triggered also by the uptake of specific molecules that may represent receptors for BMMFs. These molecules are also taken up by consumption of cow products and are metabolized into receptors on the surface of the host cells. When a certain level of antigen is reached by continuous uptake of BMMFs in combination with focal spreading of infection, the host immune response induces a state of chronic and local inflammation producing a stable increase of reactive oxygen species (ROS) and cyclooxygenase-2 (Cox-2) which dramatically increases the probability of deregulated cell proliferation with concomitant fixation of random mutations in surrounding cells induced by ROS. Especially, cells with intrinsically high replicative activity might represent targets enriching random DNA mutations enabling stochastic manifestation of mutations as a basic requirement for tumor genesis and development of prostate cancer. Thus, BMMFs represent a specific and local trigger for induction of chronic inflammation within the tissue stroma leading to an increase of ROS which induces proliferation and mutation in surrounding replicative cells eventually leading to the formation of hyperplasia as precursors for cancer.

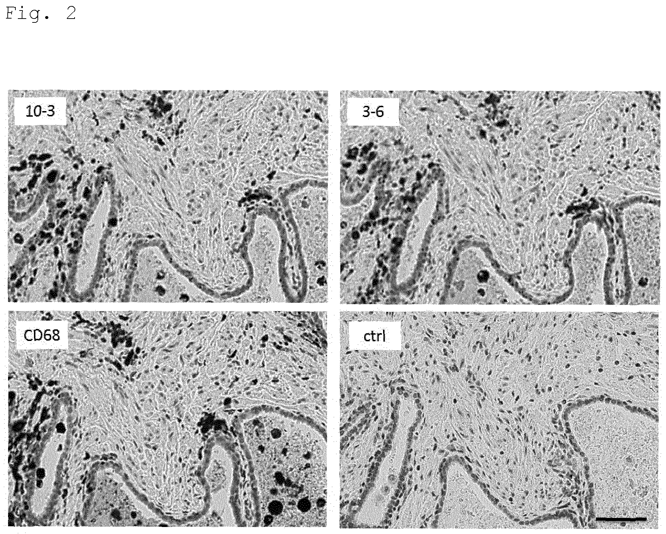

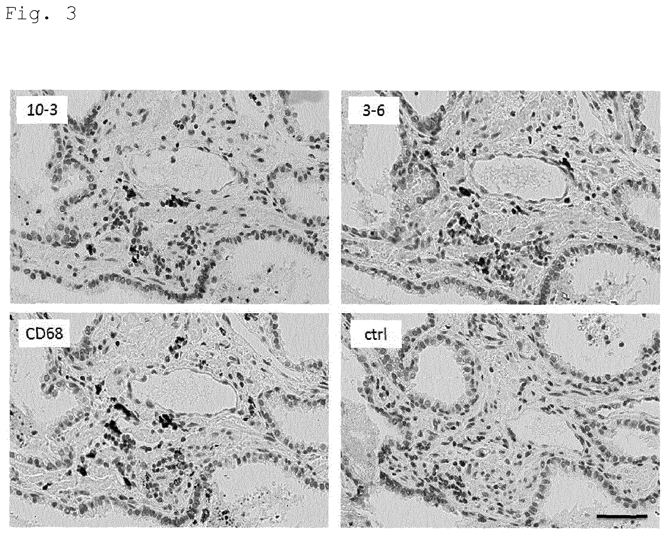

In detail, a selection of tissue samples from 12 prostate cancer patients with known tumor staging were subjected to IHC staining with mouse monoclonal anti-Rep antibodies. All tissues were tested positive for BMMF1 Rep targets. Exemplarily, the staining with anti-Rep antibodies (e.g., mAb 10-3, mAb 3-6) shows specific detection of protein targets in stromal tumor tissue regions within prostate cancer patient samples 17AD97 and 16RAV2 (Fig. 2 and 3). In general, the anti-Rep detection resulted in intense staining of smaller sized aggregates mainly within the cytoplasmic regions of cells within the stroma. Additionally, a co-localization of the anti-Rep-stained signals with CD68-positive macrophages was observed. The regions with highest Rep-specific antibody detection correlate with regions with highest detection levels for CD68-positive cells pointing towards a localization of the Rep-specific antigens in inflammatory tissue areas i.e., regions with especially high levels of inflammatory monocytes, circulating macrophages, or resident tissue macrophages. No signal detection was observed in control staining with an antibody isotype control. On the other hand, significant anti-Rep staining patterns were also observed in epithelial cells surrounding the walls of prostate ducts and acini with aggregate-like cytoplasmic localization, which might represent tissue areas enabling BMMF replication/persistence.

So far, a spectrum of 18 different, but partially related, DNA molecules were isolated from different test material (bovine sera, milk, brain tissue of one multiple sclerosis patient autopsies) (Funk et al., 2014, Gunst et al. 2014, Lamberto et al. 2014, Whitley et al. 2014; Eilebrecht et al. 2018; WO 2015/062726 A2; WO 2016/005054 A2). The 18 isolates were divided into four different groups BMMF1 through BMMF4, according to their molecular characteristics (zur Hausen et al., 2017). Three of these groups revealed a remarkable degree of similarity to *Acinetobacter baumannii* and Psychrobacter plasmids. The fourth group comprised 3 isolates being representatives of *Gemycirularviridae.* Putative Rep genes were identified as part of the BMMF's DNA sequences obtained by *in silico* comparisons to available sequences. Amplification using abutting primers in the rep gene led to the isolation of full and partial circular DNA genomes from bovine sera (Funk et al., 2014). This was extended to samples from commercially available milk products for the presence of specific circular single-stranded DNA genomes. Full-length circular single-stranded DNA molecules of 14 different isolates of (~1100 to 3000 nucleotides) were cloned and sequenced (Whitley et al., 2014; Gunst et al., 2014; Funk et al., 2014; Lamberto et al., 2014). Four additional isolates were obtained from human brain and serum (all from patients with multiple sclerosis) (Whitley et al., 2014; Gunst et al., 2014; Lamberto et al., 2014).

Among these isolates two DNA molecules closely related to transmissible spongiform encephalopathy (TSE)-associated isolate Sphinx 1.76 (1,758 bp; accession no. HQ444404 (Manuelidis L. 2011)) were isolated from brain tissue from an MS patient. These isolates were MSBI1.176 (MSBI, multiple sclerosis brain isolate) (1,766 bp) and MSBI2.176 (1,766 bp), which are designated as "MSBI1 genome" and "MSBI2 genome", respectively. MSBI1.176 shares 98% nucleotide similarity to the sequence of Sphinx 1.76. The large open reading frames (ORFs) of the isolates encode a putative DNA replication protein sharing high similarity between them. Another common feature is the presence of iteron-like tandem repeats. The alignment of this repeat region indicates a variation in the core of single nucleotides. This iteron-like repeats may constitute the binding sites for Rep proteins. The sequences of the isolates have been deposited in the EMBL Databank under accession numbers LK931491 (MSBI1.176) and LK931492 (MSBI2.176) (Whitley C. et al. 2014) and have been aligned and described in WO 2016/005054 A2.

Further isolates were obtained from cow milk. These Cow milk isolates (CMI) were CMI1.252, CMI2.214 and CMI3.168 which are designated as "CMI1 genome", "CMI2 genome" and "CMI3 genome", respectively. The sequences of the isolates have been deposited in the EMBL Databank under accession numbers LK931487 (CMI1.252), LK931488 (CMI2.214) and LK931489 (CMI3.168) and have been aligned and described in WO 2016/005054 A2.

The present inventors have found that both CMI genomes and MSBI genomes show a significant production of transcribed RNA, and the encoded Rep protein is expressed mostly in peripheral tissue around the cancer tissue. The present inventors have found that the encoded Rep proteins (MSBI1 Rep, MSBI2 Rep, CMI1 Rep, CMI2 Rep, CMI3 Rep) represent a biomarker for prostate cancer. As DNA-replication-associated protein (RepB), the Rep protein has DNA binding activity and can be essential for initiation of replication of episomal or viral DNA molecules. Rep proteins show a marked potential of self-oligomerization and aggregation, which was described within prokaryotic systems *in vivo* and *in vitro* (Giraldo et al. 2011, Torreira et al. 2015).

The present invention is defined in independent claims 1 and 2. Preferred embodiments are defined in the dependent claims.

In more detail, the present invention specifically refers to the in-vitro use of BMMF1 Rep Protein as a biomarker for prostate cancer, wherein the Rep protein is a MSBI1 genome-encoded Rep protein (MSBI1 Rep) encoded by MSBI1.176 comprising the amino acid sequence as depicted in SEQ ID NO: 1, a MSBI2 genome-encoded Rep protein (MSBI2 Rep) encoded by MSBI2.176 comprising the amino acid sequence as depicted in SEQ ID NO: 8, a CMI1 genome-encoded Rep protein (CMI1 Rep) encoded by CMI1.252 comprising the amino acid sequence as depicted in SEQ ID NO: 10, a CMI2 genome-encoded Rep protein (CMI2 Rep) encoded by CMI2.214 comprising the amino acid sequence as depicted in SEQ ID NO: 11 or a CMI3 genome-encoded Rep protein (CMI3 Rep) encoded by CMI3.168 comprising the amino acid sequence as depicted in SEQ ID NO: 12, or a variant of the protein with SEQ ID NO: 1 or SEQ ID NO: 8 having a homology of at least 90%, wherein the variant has one or more amino acid deletions, substitutions or additions and is capable of binding an anti-Rep antibody specific for a Rep protein having the amino acid sequence of SEQ ID NO: 1 or SEQ ID NO: 8.

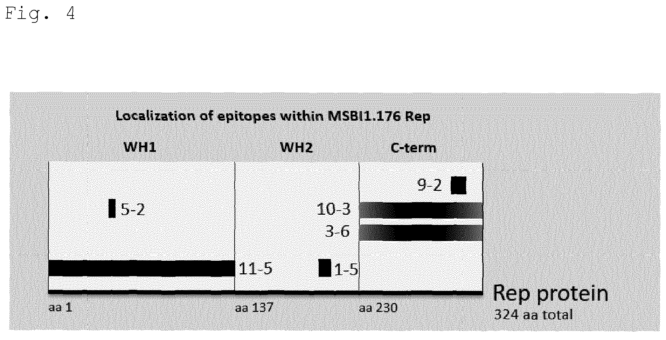

The inventors have raised monoclonal antibodies against Rep protein. In particular embodiments, the anti-Rep antibodies bind to epitopes of Rep protein that are exemplified in Fig. 4. Particularly preferred antibodies bind to epitopes within an amino acid sequence selected from the group consisting of amino acids from 1 to 136, from 137 to 229 and from 230 to 324 of SEQ ID NO: 1. For example, the antibody binds to an epitope comprised by SEQ ID NO: 2 or SEQ ID NO: 3.

### Brief description of the drawings:

- Figure 1: shows the proposed model for prostate cancer development
- Figure 2: IHC detection of BMMF1 Rep on prostate cancer patient tissue 17AD97 (scale bar = 100 µm) in consecutive tissue sections.
- Figure 3: IHC detection of BMMF1 Rep on prostate cancer patient tissue 16RAV2 (scale bar = 100 µm) in consecutive tissue sections.
- Figure 4: shows characteristics of the raised antibodies and the localization of epitopes within Rep
- Figure 5: bar diagram showing the Immunoreactive Score based on BMMF1 Rep staining (X-axis: Immunoreactive Score; Y-axis: number of patients)

The invention provides the teaching that Rep proteins may represent biomarkers for an enhanced risk to develop prostate cancer and are useful as a marker for determining the overall survival prognosis of prostate cancer patients.

The term "prostate cancer" means a malignant tumor that evolved as a consequence of uncontrolled cell growth in the prostate. These malignancies may develop as a consequence of pre-existing benign hyperplasia where genetic alterations promote the transition from normal to cancerous growth. The term "prostate cancer" means pre-stages, early stages or late stages of the disease and metastases derived therefrom.

In an alternative embodiment, the present invention may also encompass the systematic testing of healthy prostate tissue (tissue from individuals without cancer diagnosis or a specific hint for the disease) to assess the disease risk in the future.

This means that the present invention is also suitable to determine the predisposition for developing prostate cancer.

"Rep protein" as used herein refers to a DNA-replication-associated protein (RepB). The Rep protein comprises DNA binding activity and could be essential for initiation of replication of episomal/viral DNA molecules. In general Rep protein refers to a Rep protein from the group of the Small Sphinx Genome (Whitley et al., 2014). In accordance with the present invention, the Rep protein is a MSBI1 genome-encoded Rep protein (MSBI1 Rep), a MSBI2 genome-encoded Rep protein (MSBI2 Rep), a CMI1 genome-encoded Rep protein (CMI1 Rep), a CMI2 genome-encoded Rep protein (CMI2 Rep) or a CMI3 genome-encoded Rep protein (CMI3 Rep) as encoded by the sequences exemplified in the appended set of claims.

According to the present invention, the MSBI1 Rep protein is encoded by MSBI1.176 deposited in the EMBL databank under the acc. no. LK931491 and has the amino acid sequence as depicted in SEQ ID NO: 1, the Rep protein is MSBI2 encoded by MSBI2.176 deposited in the EMBL databank under the acc. no. LK931492 and has the amino acid sequence as depicted in SEQ ID NO: 8 (Whitley, Gunst et al. 2014) or the CMI1 Rep protein is encoded by CMI1.252 deposited in the EMBL databank under the acc. no. LK931487 and has the amino acid sequence as depicted in SEQ ID NO: 10, the CMI2 Rep protein is encoded by CMI2.214 deposited in the EMBL databank under the acc. no. LK931488 and has the amino acid sequence as depicted in SEQ ID NO: 11, or the CMI3 Rep protein is encoded by CMI3.168 deposited in the EMBL databank under the acc. no. LK931489 and has the amino acid sequence as depicted in SEQ ID NO: 12.

In a particular preferred embodiment, the Rep protein comprises a N-terminal region conserved among BMMF1 genomes consisting essentially of amino acids from 1 to 229 of SEQ ID NO: 1 and a C-terminal variable region specific for MSBI1.176 consisting essentially from amino acids 230 to 324 of SEQ ID NO:1. The N-terminal conserved region comprises a putative, first DNA binding domain consisting essentially of amino acids from 1 to 136 of SEQ ID NO: 1 and a second putative DNA binding domain consisting essentially of amino acids from 137 to 229 of SEQ ID NO: 1. The C-terminal domain shows little sequence homology with any known protein and consists of amino acids 230 to 324.

The "Rep protein" of the present invention also encompasses fragments and variants of the protein with SEQ ID NO: 1 or SEQ ID NO: 8 which are capable of binding an anti-Rep antibody specific for Rep protein having the amino acid sequence of SEQ ID NO: 1 or SEQ ID NO: 8. Preferably, such a fragment is an immunogenic fragment of the protein having the amino acid sequence of SEQ ID NO: 1 or SEQ ID NO: 8 which encompasses at least one epitope for an anti-Rep protein antibody against the Rep protein of SEQ ID NO: 1 or SEQ ID NO: 8 and, preferably, comprises at least 7, 8, 9, 10, 15, 20, 25 or 50 contiguous amino acids. In particular embodiments, the fragment comprises or consists essentially of a domain of the Rep protein, for example, the N-terminal conserved region, the C-terminal variable region, the first or second DNA binding domain. A variant of the protein with SEQ ID NO: 1 or SEQ ID NO: 8 comprises one or more amino acid deletions, substitutions or additions compared to SEQ ID NO: 1 and has a homology of at least 90%, 91%, 92%, 93%, 94%, 95%, 96%, 97%, 98% or 99% to the amino acid sequence of SEQ ID NO: 1 or SEQ ID NO: 8, wherein the variant is capable of binding an anti-Rep antibody specific for a Rep protein having the amino acid sequence of SEQ ID NO: 1 or SEQ ID NO: 8. Included within the definition of variant are, for example, polypeptides containing one or more analogues of an amino acid (including, for example, unnatural amino acids, peptide nucleic acid (PNA), etc.), polypeptides with substituted linkages, as well as other modifications known in the art, both naturally occurring and non-naturally occurring.

The term Rep protein includes fusion proteins with a heterologous amino acid sequence, with a leader sequence or with a Tag-sequence and the like. In certain embodiments of the invention protein tags are genetically grafted onto the Rep protein described above i.e., the Rep protein selected from the group consisting of MSBI1, MSBI2, CMI1, CMI2 or CMI3 as encoded by the sequences exemplified in the appended set of claims. In particular at least one protein tag is attached to a polypeptide consisting of an amino acid sequence as depicted in any one of SEQ ID NOs: 1-3, 8, 10-12. Such protein tags may be removable by chemical agents or by enzymatic means. Examples of protein tags are affinity or chromatography tags for purification. For example, the Rep protein may be fused to a Tag-sequence, for example, selected from the group consisting of His₆-Tag (SEQ ID NO: 4), T7-Tag (SEQ ID NO: 5), FLAG-Tag (SEQ ID NO: 6) and Strep-II-Tag (SEQ ID NO: 7), an His-Tag (SEQ ID No: 4), a T7-Tag (SEQ ID NO: 5), FLAG-Tag (SEQ ID NO: 6) or Strep-II-Tag (SEQ ID NO: 7). Further, fluorescence tags such as green fluorescence protein (GFP) or its variants may be attached to a Rep-protein according to the invention.

In general, the MSBI1 genome-encoded Rep protein (MSBI1 Rep) may be codon-optimized (e.g. SEQ ID NO: 14) for the production in human cell lines (e.g., HEK-293, HEK293TT, HEK293T, HEK293FT, HaCaT, HeLa, SiHa, CaSki, HDMEC, L1236, L428, BJAB, MCF7, Colo678, any primary cell lines) as well as bovine (e.g., MAC-T) or murine cell lines (e.g., GT1-7). This is described in detail in PCT/EP2017/075774.

The Rep protein of the invention, including the Rep fragments and Rep variants as defined above, can be prepared by classical chemical synthesis. The synthesis can be carried out in homogeneous solution or in solid phase. The polypeptides according to this invention can also be prepared by means of recombinant DNA techniques.

"Subject" as used herein refers to a mammalian individual or patient, including murines, cattle, for example bovines, simians and humans. Preferably, the subject is a human patient.

"Anti-Rep antibody" as used herein refers to an antibody binding at a detectable level to Rep protein which affinity is more strongly to the Rep protein of the invention than to a non-Rep protein. Preferably, the antigen affinity for Rep protein is at least 2-fold larger than background binding. In particular, the anti-Rep antibody is specific for the MSBI1 Rep having the amino acid sequence of SEQ ID NO: 1 or MSBI2 Rep having the amino acid sequence of SEQ ID NO: 8. In particular embodiments, the antibody is cross-specific for MSBI1 Rep, MSBI2 Rep, CMI1 Rep, CMI2 Rep and/or CMI3 Rep comprising the sequences of the present invention. In certain embodiments the anti-Rep antibody is cross-specific for at least two, preferably all, off MSBI1 Rep, MSBI2 Rep, CMI1 Rep, CMI2 Rep and/or CMI3 Rep comprising the sequences of the present invention, which are exemplified in the appended set of claims.

The inventors also tested the antibody level of prostate cancer patients by contacting the Rep protein with a specimen suspected of containing anti-Rep protein antibodies under conditions that permit the Rep protein to bind to any such antibody present in the specimen. Such conditions will typically be physiologic temperature, pH and ionic strength using an excess of Rep protein. The incubation of the Rep protein with the specimen is followed by detection of immune complexes comprised of the antigen. In certain embodiments either the Rep protein is coupled to a signal generating compound e.g., detectable label, or an additional binding agent e.g., secondary antihuman antibody, coupled to a signal generating compound is used for detecting the immune complex.

Anti-Rep antibodies can be detected and quantified in assays based on Rep protein as protein antigen, which serves as target for the mammalian e.g., human, antibodies suspected in the specimen. Preferably, the Rep protein is purified, and the specimen can be, for example, serum or plasma. The methods include immobilization of Rep protein on a matrix followed by incubation of the immobilized Rep protein with the specimen. Finally, the Rep-bound antibodies of the formed immunological complex between Rep protein and antibodies of the specimen are quantified by a detection binding agent coupled to a signal generating compound e.g., secondary HRP-(horseradish-peroxidase)-coupled detection antibody allowing for HRP-substrate based quantification. This signal generating compound or label is detectable in itself or may be reacted with an additional compound to generate a detectable product.

Design of the immunoassay is subject to a great deal of variation, and many formats are known in the art. Protocols may, for example, use solid supports, or immunoprecipitation. Most assays involve the use of binding agents coupled to signal generating compounds, for example labelled antibody or labelled Rep protein; the labels may be, for example, enzymatic, fluorescent, chemiluminescent, radioactive, or dye molecules. Assays which amplify 8the signals from the immune complex are also known; examples of which are assays which utilize biotin and avidin or streptavidin, and enzyme-labelled and mediated immunoassays, such as ELISA assays.

The immunoassay may be in a heterogeneous or in a homogeneous format, and of a standard or competitive type. Both standard and competitive formats are known in the art.

In an immunoprecipitation or agglutination assay format the reaction between the Rep protein and the anti-Rep antibody forms a network that precipitates from the solution or suspension and forms a visible layer or film of precipitate. If no anti-Rep antibody is present in the specimen, no visible precipitate is formed.

In further embodiments the inventors used methods wherein an increased amount of Rep protein in a sample correlates with a diagnosis or predisposition of prostate cancer. In such embodiments the Rep protein in the sample is detected by anti-Rep antibodies.

"Sample" as used herein refers to a biological sample encompassing cancerous prostate tissue, peripheral tissue surrounding the cancerous tissue and (benign) hyperplasia. The samples encompass tissue samples such as tissue cultures or biopsy specimen.

Such methods (ex-vivo/in-vitro methods) comprise the steps of detecting Rep protein in a sample from a subject by anti-Rep antibodies. In such methods Rep protein is detected in tissue samples by immunohistochemical methods or immunofluorescence microscopy.

In certain embodiments anti-Rep antibodies are used for the detection or capturing of the Rep protein in the sample.

The term "antibody" preferably relates to antibodies which consist essentially of pooled polyclonal antibodies with different epitope specificities, as well as distinct monoclonal antibody preparations. As used herein, the term "antibody" (Ab) or "monoclonal antibody" (Mab) is meant to include intact immunoglobulin molecules as well as antibody fragments (such as, for example, Fab and F(ab')2 fragments) which are capable of specifically binding to Rep protein. Fab and F(ab')2 fragments lack the Fc fragment of intact antibody, clear more rapidly from the circulation, and may have less non-specific tissue binding than an intact antibody. Thus, these fragments are preferred, as well as the products of a FAB or other immunoglobulin expression library. Moreover, antibodies useful for the purposes of the present invention include chimeric, single chain, multifunctional (e.g., bispecific) and humanized antibodies or human antibodies.

In certain embodiments the antibody or antigen binding fragment thereof is coupled to a signal generating compound, e.g., carries a detectable label. The antibody or antigen binding fragment thereof can be directly or indirectly detectably labelled, for example, with a radioisotope, a fluorescent compound, a bioluminescent compound, a chemiluminescent compound, a metal chelator or an enzyme. Those of ordinary skill in the art will know of other suitable labels for binding to the antibody, or will be able to ascertain such, using routine experimentation.

The anti-Rep antibodies of the present invention are raised (generated) against a Rep protein having the amino acid sequence of SEQ ID NO: 1 or SEQ ID NO: 8 or a fragment thereof by methods well known to those skilled in the art.

In certain embodiments anti-Rep antibodies are used in the methods of the invention which are capable of binding to several or all kinds of Rep proteins from the group of the Small Sphinx Genome (anti-Small-Sphinx-like Rep antibody or anti-SSLRep antibody). Such anti-SSLRep antibody binds to an epitope within the conserved N-terminal region of the Rep protein from amino acids 1 to 229 of SEQ ID NO: 1. In particular embodiments anti-Rep antibodies of the anti-SSLRep type are used which bind to an epitope within SEQ ID NO: 2 (amino acids 32-49 of SEQ ID NO:1) or SEQ ID NO: 3 (amino acids 197-216 of SEQ ID NO: 1). The peptide fragments of SEQ ID NO: 2 and SEQ ID NO: 3 are highly conserved among the Rep proteins from the Small Sphinx Genome group and appear to be exposed due to their hydrophilic character. Anti-Rep antibodies of the anti-SSLRep type may be produced by immunization, for example of mice or guinea pig, by peptides consisting essentially of the amino acid sequences as depicted in SEQ ID NOs: 2 or 3; or by other immunogenic fragments, preferably comprising at least 8-15 amino acids, derived from the conserved N-terminal Rep protein region from amino acids 1 to 229 of SEQ ID NO: 1.

In further embodiments, anti-Rep antibodies specific for MSBI1 Rep protein are used. Such antibodies may be produced, for example, by immunization of a mammal such as mice or guinea pig with a full-length Rep protein having the amino acid sequence of SEQ ID NO: 1.

Preferably, the methods of the invention use anti-Rep antibodies, which are capable of detecting Rep protein up to ranges from picogram to femtogram.

Examples of such groups of anti-Rep antibodies are shown in Table 1:

| **Antibody Group** | **Rep-Protein Localisation** | **Specificity** | **Antibody** | **DSMZ deposit** |
|---|---|---|---|---|
| **Group A** | cytoplasm + nuclear membrane (+nucleus) | MSBI1 + small-sphinx-like | AB01 523-1-1 (Ab 1-5) | DSM ACC3327 |
| | | All BMMF1 Reps | | |
| **Group B** | speckles in cytoplasm | MSBI1 + small-sphinx-like | AB02 304-4-1 (Ab 5-2) | DSM ACC3328 |
| **Group C** | cytoplasm + nuclear membrane (+ nucleus) | MSBI1 specific | MSBI1 381-6-2 (Ab 3-6) | DSM ACC3329 |
| | | | MSBI1 572-13-19 (Ab 10-3) | |
| | | | MSBI1 617-1-3 (Ab 11-5) | |
| **Group D** | speckles in cytoplasm | MSBI1 specific | D1: MSBI1 961-2-2 (Ab 9-2) | DSM ACC3331 |
| | | | D2: MSBI1 761-5-1 (Ab 13) | DSM ACC3330 |

Anti-Rep antibodies of group A have an epitope within the amino acid sequence depicted in SEQ ID NO: 3 (aa 198-217 of SEQ ID NO: 1) and are capable of detecting MSBI1 Rep and Rep proteins comprising this conserved epitope of the Small Sphinx Genome group (e.g., MSBI2, CMI1, CMI4). In immunofluorescence assays such anti-Rep antibodies detect a specific Rep localisation pattern, wherein the main localisation is homogeneously distributed over the cytoplasm and nuclear membrane; and additional weak and homogeneously distributed localisation is seen in the nucleus. An example of such a group A antibody is antibody AB01 523-1-1 (also called antibody 1-5; DSM ACC3327) which was employed in the examples as group A antibody.

Anti-Rep antibodies of group B have an epitope within the amino acid sequence depicted in SEQ ID NO: 2 (aa 33-50 of SEQ ID NO: 1) and are capable of detecting MSBI1 Rep and Rep proteins comprising this conserved epitope of the Small Sphinx Genome group (e.g., MSBI2, CMI1, CMI4). In immunofluorescence assays such anti-Rep antibodies detect specifically speckles (cytoplasmatic aggregations) of the Rep protein (often in the periphery of the nuclear membrane). An example of such a group B antibody is the antibody designated as AB02 304-4-1 (also called antibody 5-2; DSM ACC3328) which was employed in the examples as group B antibody.

Anti-Rep antibodies of group C detect specifically a structural epitope of MSBI1 (SEQ ID NO: 1). In immunofluorescence assays such anti-Rep antibodies detect a specific Rep localisation pattern, wherein the main localisation is homogeneously distributed over the cytoplasm and nuclear membrane; and additional weak and homogeneously distributed localisation is seen in the nucleus. An example of such a group C antibody is antibody MSBI1 381-6-2 (also called antibody 3-6; DSM ACC3329) which was employed in the Example as group C antibody with an epitope in the sequence of aa 230-324. Another example of an antibody of a group C antibody is antibody MBSI1 572-13-19 (also called antibody 10-3) detecting an epitope in the C-terminal domain of MSBI 1 Rep (aa 230-324). Another example of an antibody of a group C antibody is antibody MBSI1 617-1-3 (also called antibody 11-5) detecting an epitope in the N-terminal domain of MSBI1 Rep (aa 1-136).

Anti-Rep antibodies of group D detect specifically a structural epitope of MSBI1 (SEQ ID NO: 1), where antibody MSBI1 961-2-2 designated as "D1" (also called antibody 9-2; DSM ACC3331) detects an epitope depicted in SEQ ID NO: 9 (aa 281-287) in the C-terminal domain of MSBI1. Antibody MSBI1 761-5-1 (also called antibody 13; DSM ACC3328) designated as "D2" detects a 3D structural epitope of MSBI1 which is exclusively accessible under in-vivo conditions and is not accessible in Western Blots. In immunofluorescence assays such anti-Rep antibodies detect specifically speckles (cytoplasmatic aggregations) of the Rep protein (often in the periphery of the nuclear membrane.

The invention is further illustrated by the following examples:

### Example 1: Detection of BMMF protein targets in prostate tissue

All tissue samples were provided by the tissue bank of the National Center for Tumor Diseases (NCT, Heidelberg, Germany and Institute of Pathology, Heidelberg University Hospital, Germany) in accordance with the regulations of the tissue bank and the approval of the ethics committee of Heidelberg University.

### Tissue staining

The paraffin-embedded tissue sections (~4 µm thickness) were stained with the Zytomed Chem-Plus HRP Polymer-Kit (Zytomed, POLHRP-100) and the DAB Substrate Kit High Contrast (Zytomed, DAB500plus) after EDTA epitope retrieval (Sigma E1161) with the given antibody incubations (c.f. Table 1) and hematoxylin counterstain. Slides were scanned with a digital slide scanner (Hamamatsu) and analyzed based on with NDPview2 Plus software (Hamamatsu).

**Table 1**

| **Antibody** | **Source** | **Host** | **Dilution** | **Final concentration in µg/ml** | **Incubation time** |
|---|---|---|---|---|---|
| **Primary** | | | | | |
| Rep mAb #3-6 | T. Bund, DKFZ | mouse | 1:500 | 3.9 | 30 min at room temperature |
| Rep mAb #10-3 | T. Bund, DKFZ | mouse | 1:500 | 3.9 | |
| CD68 | Cell signaling #76437 | rabbit | 1:1000 | | |

| **Secondary** | | | | | |
|---|---|---|---|---|---|
| rabbit anti-mouse | Abcam #125904 | rabbit | 1:500 | | 20 min at room temperature |

Staining with anti-Rep antibodies (e.g., mAb 10-3, mAb 3-6) shows specific detection of protein targets in stromal tumor tissue regions within prostate cancer patient samples 17AD97 and 16RAV2 (Fig. 2 and 3). In general, the anti-Rep detection resulted in intense staining of smaller sized aggregates mainly within the cytoplasmic regions of cells within the stroma. Additionally, a co-localization of the anti-Rep-stained signals with CD68-positive macrophages was observed. The regions with highest Rep-specific antibody detection correlate with regions with highest detection levels for CD68 positive cells pointing towards a localization of the Rep-specific antigens in inflammatory tissue areas i.e., regions with especially high levels of inflammatory monocytes, circulating macrophages, or resident tissue macrophages. No signal detection was observed in control staining with an antibody isotype control.

### Example 2: Tissue Staining and Tissue Analysis

Tissue microarray TMA105 was generated and provided by courtesy of NCT Heidelberg. In this data set each 4 tumoral tissues were available for a total number of 120 patients and each 2 peritumoral tissue spots for a total number of 14 patients.

TMA 105 was stained fully automatically on a BOND MAX machine (Leica Biosystems) with EDTA epitope retrieval buffer (Abcam, #ab93680). Primary antibody anti-BMMF1 Rep (#3-6, monoclonal, DKFZ Heidelberg) and isotype control antibody (Biolegend IgG1, MG1-45) were incubated for 30 min at room temperature (4 µg/ml). Secondary rabbit anti-mouse (Abcam #125904) was incubated for 20 min at room temperature. Detection was performed by using Bond Polymer Refine Detection Kit (Leica #DS9800) including DAB chromogen and hematoxylin counterstain. Slides were scanned using a Hamamatsu Nanozoomer slide scanner (Hamamatsu) and analyzed with NDP.view2 Plus software (Hamamatsu).

### Tissue analysis

For analysis of BMMF1 Rep staining on the TMAs, the antibody staining was characterized based on two parameters: the percentage of stained cells (positivity) and intensity (I) of the signal within interstitial/stromal parts of the tissue spots. Epithelial parts and tumor cells were not included into analysis as they are not the target of BMMF positivity, in general. The positivity (POS) of BMMF1 Rep staining was assessed using a three-level scale in which 0 indicated no positive tissue parts at all, 1 indicated 1-10% positive, 2 indicated 11-30%, 3 indicated more than 30% positive cells distributed in several regions of the tissue spot. Intensity (I) was graded as follows: 0 = no detection, 1 = moderate, 2 = intense staining. For statistical analysis, the immunoreactive score (IRS) was calculated as follows: IRS= I x POS; minimum value=0, maximum value=6 (Tab. 2).

**Table 2: Scoring parameters for quantification of BMMF1 Rep staining on TMAs.**

| **Target** | **Intensity (Staining intensity, I)** | **I** | **Positivity (proportion of positive cells, POS)** | **POS** |
|---|---|---|---|---|
| **BMMF1 Rep** | 0 | no detection | 0 | 0 |
| | 1 | moderate | 1 | 1-10% |
| | 2 | strong | 2 | 11-30% |
| | | | 3 | >31% |

IRS = I x POS
IRS= immunoreactive score

Using these scoring criteria the samples from tumor tissue (120 patients) based on BMMF1 Rep staining are:
12% negative (IRS 0)
88% positive (at least IRS 1) [with 75% significantly positive = at least IRS 2]

The samples from peritumoral tissue (14 patients) based on BMMF1 Rep staining are:
29% negative (IRS 0)
71% positive (at least IRS 1) [with 21% significantly positive = at least IRS 2]

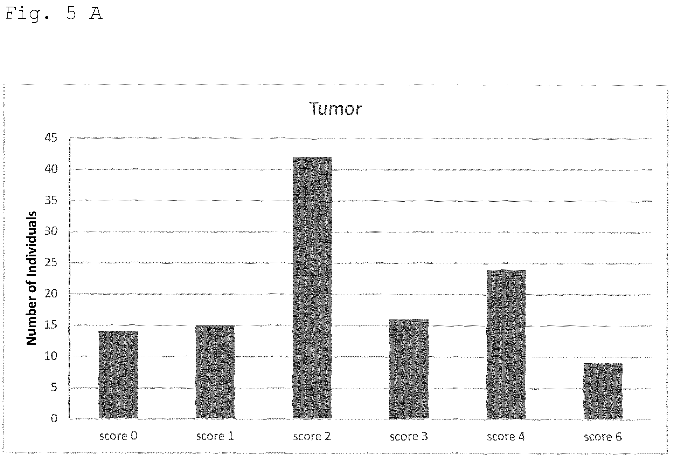

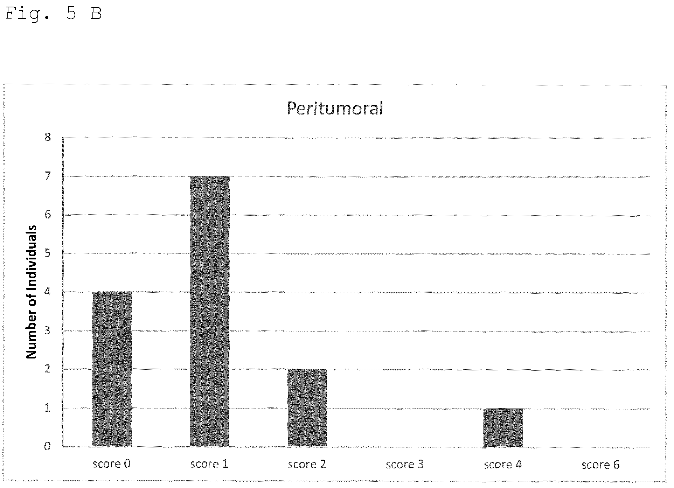

These results are shown as bar diagrams in Figs. 5 A and B.

### SEQUENCE SUMMARY

| SEQ ID NO | SEQUENCE |
|---|---|
| 1 | Amino acid sequence of Rep protein encoded by MSBI1.176 |
| | MSDLIVKDNALMNASYNLALVEQRLILLAIIEARETGKGINANDPLTVHASSYINQFN VERHTAYQALKDACKDLFARQFSYQEKRERGRINITSRWVSQIGYMDDTATVEIIFAP AVVPLITRLEEQFTQYDIEQISGLSSAYAVRMYELLICWRSTGKTPIIELDEFRKRIG VLDTEYTRTDNLKMRVIELALKQINEHTDITASYEQHKKGRVITGFSFKFKHKKQNSD KTPKNSDSSPRIVKHSQIPTNIVKQPENAKMSDLEHRASRVTGEIMRNRLSDRFKQGD ESAIDMMKRIQSEIITDAIADQWESKLEEFGVVF |
| 2 | Amino acid sequence of Rep peptide fragment |
| | EARETGKGINANDPLTVH |
| 3 | Amino acid sequence of Rep peptide fragment |
| | KQINEHTDITASYEQHKKGRT |
| 4 | His-Tag (with two neutral stuffer amino acids) |
| | GAHHHHHH |
| 5 | T7-Tag |
| | MASMTGGQQMG |
| 6 | FLAG-Tag |
| | DYKDDDDK |
| 7 | Strep-II-Tag |
| | WSHPQFEK |
| 8 | MSKLVVKDNALMNASYNLDLVEQRLILLAIIEARESGKGINANDPLTVHA ESYINQFGVHRVTAYQALKDACDNLFARQFSYQSKSEKGNIQNHRSRWVS EIIYIDTEATVKIIFAPAIVPLITRLEEQFTKYDIEQISDLSSAYAIRLY ELLICWRSTGKTPIIGLGEFRNRVGVLDSEYHRIAHLKERVIEHSIKQIN EHTDITATYEQHKKGRTITGFSFKFKQKKPKQAEIATETPKTATNDPDTT KPLTEPQIAKYSMILCKLGSISDLSNFPDYPAFANWIGNILRNPEKADEQ IAKRIFTALKTETDYSKKN Amino acid sequence of Rep protein encoded by MSBI2.176 |
| 9 | MSBI.1 specific epitope |
| | NRLSDRF |
| 10 | Amino acid sequence of Rep protein encoded by CMI1.252 |
| | MSDLIVKDNALMNASYNLALVEQRLILLAILEARETGKGINANDPLTVHASSYINQFN VERHTAYQALKDACKDLFARQFSYQEKRERGRINITSRWVSQIGYMDDTATVEIIFAP AVVPLITRLEEQFTQYDIEQISELSSAYAVRLYELLICWRSTGKTPIIDLTEFRKRLG VLDTEYTRTDNLKMRVIELGLKQINEHTDITASYEQHKKGRTITGFSFKFKQKKKTGA EMPKNSDSSPHIEKPSQIPANIAKQPENAKKDDLGHRASKITGLIMSNGLADRFKRGD ESVIDMMKRIKEEITTDTTADQWENKLEEFGVIFQS |
| 11 | Amino acid sequence of Rep protein encoded by CMI2.214 |
| | MSDLIVKDNALMNASYNLDLVEQRLILLAILEARETGKGINANDPLTVHAESYINQFG VARQTAYQALKDACKDLFARQFSYQEKRERGRANITSRWVSQIAYIDETATVEVIFAP AVVPLITRLEEQFTQYDIEQISGLSSAYAVRLYELLICWRSTGKTPVIELAEFRKRLG VLNDEYTRSDNFKKWIIENPIKQINEHTDITASYEQHKKGRTITGFSFKFKQKKKTEP ETPKNSDSSQRIEKPSQIPANIVKQPENANLSDLQHRASKITGLIMSNRLSDRFKQGD ESIMQMMARIQSEITTDSIADQWQSKLEEFGVVF |
| 12 | Amino acid sequence of Rep protein encoded by CMI3.168 |
| | MSDLIVKDNALMNASYNLALVEQRLILLAILEARETGKGINANDPLTVHASSYINQFN VERHTAYQALKDACKDLFARQFSYQEKRERGRANITSRWVSQIAYIDETATVEVIFAP AVVPLITRLEEQFTQYDIEQISGLSSAYAVRLYELLICWRTTGKTPVLDLTEFRKRLG VLDTEYTRTDNLKMRVIEQSLKQINKHTDITASYEQHKKGRTITGFSFKFKQKKKTEP ETPKNNDSGVSKPKTVEIPAEVVKQPKNTNLSDLEKRVRMITGAIAKNNLASRFQHGN ESPLDMMKRIQSEITSDETADLWQNKLESMGVVF |
| 13 | DNA sequence MSBI1 Rep codon-optimized |
| | ATGAGCGACCTGATCGTGAAAGACAATGCCCTGATGAACGCCTCCTACAACCTGGCAC TGGTCGAACAGAGACTGATTCTGCTGGCTATCATCGAGGCAAGGGAGACCGGCAAGGG CATCAACGCCAATGACCCCCTGACAGTGCACGCCAGCTCCTACATCAACCAGTTTAAT GTGGAGCGCCACACCGCCTATCAGGCCCTGAAGGACGCCTGCAAGGATCTGTTTGCCC GGCAGTTCAGCTACCAGGAGAAGCGGGAGAGAGGCAGGATCAACATCACAAGCAGATG GGTGTCCCAGATCGGCTATATGGACGATACCGCCACAGTGGAGATCATCTTTGCACCA GCAGTGGTGCCTCTGATCACCAGGCTGGAGGAGCAGTTCACACAGTACGACATCGAGC AGATCTCCGGACTGTCTAGCGCCTACGCCGTGCGCATGTATGAGCTGCTGATCTGTTG GCGGTCTACCGGCAAGACACCTATCATCGAGCTGGATGAGTTCCGCAAGCGGATCGGC GTGCTGGACACCGAGTACACCAGAACAGATAACCTGAAGATGAGAGTGATCGAGCTGG CCCTGAAGCAGATCAATGAGCACACCGATATCACAGCCTCTTATGAGCAGCACAAGAA GGGCCGCGTGATCACCGGCTTCAGCTTTAAGTTCAAGCACAAGAAGCAGAACTCTGAC AAGACACCAAAGAATAGCGATTCCTCTCCCCGGATCGTGAAGCACAGCCAGATCCCTA CCAACATCGTGAAGCAGCCAGAGAATGCCAAGATGTCCGACCTGGAGCACAGGGCATC TAGGGTGACAGGCGAGATCATGAGAAATAGGCTGAGCGATCGGTTCAAGCAGGGCGAC GAGTCCGCCATCGATATGATGAAGAGAATCCAGTCCGAGATCATCACCGACGCCATCG CCGATCAGTGGGAATCTAAACTGGAAGAGTTTGGAGTCGTGTTTGGAGCACATCACCA TCATCATCACTGA |
| 14 | Protein sequence MSBI1 Rep codon-optimized |
| | MSDLIVKDNALMNASYNLALVEQRLILLAIIEARETGKGINANDPLTVHASSYINQFN VERHTAYQALKDACKDLFARQFSYQEKRERGRINITSRWVSQIGYMDDTATVEIIFAP AVVPLITRLEEQFTQYDIEQISGLSSAYAVRMYELLICWRSTGKTPIIELDEFRKRIG VLDTEYTRTDNLKMRVIELALKQINEHTDITASYEQHKKGRVITGFSFKFKHKKQNSD KTPKNSDSSPRIVKHSQIPTNIVKQPENAKMSDLEHRASRVTGEIMRNRLSDRFKQGD ESAIDMMKRIQSEIITDAIADQWESKLEEFGVVFGA |
| 15 | DNA sequence MSBI1 Rep wild-type |
| | ATGAGCGATTTAATAGTAAAAGATAACGCCCTAATGAATGCTAGTTATAACTTAGCTT TGGTTGAACAGAGGTTAATTCTATTAGCAATCATAGAAGCGAGAGAAACAGGCAAAGG GATTAATGCCAATGATCCTTTAACAGTTCATGCAAGTAGCTATATCAATCAATTTAAC GTAGAAAGGCATACGGCATATCAAGCCCTCAAAGATGCTTGTAAAGACTTGTTTGCCC GTCAATTCAGTTACCAAGAAAAGCGAGAACGAGGACGAATTAATATTACAAGTCGATG GGTTTCGCAAATTGGCTATATGGACGATACAGCAACCGTTGAGATTATTTTTGCCCCT GCGGTTGTTCCTCTGATTACACGGCTAGAGGAACAGTTCACCCAGTACGATATTGAGC AAATTAGCGGTTTATCGAGTGCATATGCTGTTCGTATGTACGAACTGCTGATTTGTTG GCGTAGCACAGGCAAAACACCAATTATTGAGCTAGACGAGTTTAGAAAGCGAATAGGT GTTTTAGATACTGAATACACTAGAACAGATAATTTAAAGATGCGAGTTATTGAATTAG CCCTAAAACAAATCAACGAACATACAGACATCACAGCAAGCTATGAACAACACAAAAA AGGGCGAGTGATTACAGGATTCTCATTCAAGTTTAAGCACAAGAAACAAAACAGCGAT AAAACGCCAAAAAATAGCGATTCTAGCCCACGTATCGTAAAACATAGTCAAATCCCTC CAACATTGTAAAACAGCCTGAAAACGCCAAAATGAGCGATTTAGAACATAGAGCGAGC CGTGTTACAGGGGAAATAATGCGAAATCGTCTGTCAGATCGGTTTAAACAAGGCGATG AATCAGCAATCGACATGATGAAACGTATTCAAAGTGAAATAATAACCGATGCAATAGC AGACCAGTGGGAAAGCAAACTGGAGGAGTTTGGCGTGGTTTTTTAG |

### REFERENCES:

Eilebrecht, S., et al. (2018). "Expression and replication of virus-like DNA in human cells", Scientific Reports 8:2851.
Funk, M., et al. (2014). "Isolation of protein-associated circular DNA from healthy cattle serum". Genome Announc 2(4).
Giraldo, R., et al. (2011). "RepA-WH1 prionoid: a synthetic amyloid proteinopathy in a minimalist host." Prion 5(2):60-64.
Gollapudi, K., et al. (2013). "Association between tumor-associated macrophage infiltration, high grade prostate cancer, and biochemical recurrence after radical prostatectomy." Am J Cancer Res 3(5):523-529.
Gunst, K., et al. (2014). "Isolation of bacterial plasmid-related replication-associated cirular DNA from a serum sample of a multiple sclerosis patient." Genome Announc 2(4).
Lamberto, I., et al. (2014). "Mycovirus-like DNA virus sequences from cattle serum and human brain and serum samples from multiple sclerosis patients." Genome Announc 2(4).
Lippi, G. et al. (2015), Critical Reviews in Oncology/Hematology, 97:1-14.
Manuelidis L., 2011. "Nuclease resistant circular DNAs co-purify with infectivity in scrapie and CJD". J. Neurovirol. 17:131-145.
Torreira, E., et al. (2015). "Amyloidogenesis of bacterial prionoid RepA-WH1 recaptiulates dimer to monomer transitions of RepA in DNA replication initiation." Structure 23(1):183-189.
Whitley, C., et al. (2014). "Novel replication-competent cirulara DNA molecules from healthy cattle serum and milk and multiple sclerosis-affected human brain tissue." Genome Announc 2(4).
Zur Hausen, H., Bund, T., de Villiers, E.-M. (2017). "Infectious agents in bovine red meat and milk and their potential role in cancer and other chronic diseases." Curr. Top. Microbiol. Immunol., Volume 407, 83-116.

## Claims

1. An in-vitro use of Bovine Meat and Milk Factor Group 1 (BMMF1) Rep Protein as a biomarker for prostate cancer, wherein the Rep protein is a MSBI1 genome-encoded Rep protein (MSBI1 Rep) encoded by MSBI1.176 comprising the amino acid sequence as depicted in SEQ ID NO: 1, a MSBI2 genome-encoded Rep protein (MSBI2 Rep) encoded by MSBI2.176 comprising the amino acid sequence as depicted in SEQ ID NO: 8, a CMI1 genome-encoded Rep protein (CMI1 Rep) encoded by CMI1.252 comprising the amino acid sequence as depicted in SEQ ID NO: 10, a CMI2 genome-encoded Rep protein (CMI2 Rep) encoded by CMI2.214 comprising the amino acid sequence as depicted in SEQ ID NO: 11, a CMI3 genome-encoded Rep protein (CMI3 Rep) encoded by CMI3.168 comprising the amino acid sequence as depicted in SEQ ID NO: 12, or a variant of the protein with SEQ ID NO: 1 or SEQ ID NO: 8 having a homology of at least 90%, wherein the variant has one or more amino acid deletions, substitutions or additions and is capable of binding an anti-Rep antibody specific for a Rep protein having the amino acid sequence of SEQ ID NO: 1 or SEQ ID NO: 8.

2. An in-vitro method for providing a diagnosis or predisposition for prostate cancer in a subject comprising the step of detecting Rep protein in a sample from a subject by anti-Rep antibodies, wherein the anti-Rep antibodies are specific for MSBI1 Rep, MSBI2 Rep, CMI1 Rep, CMI2 Rep and/or CMI3 Rep according to claim 1, or for the variant of the protein with SEQ ID NO: 1 or SEQ ID NO: 8 having a homology of at least 90%, wherein the variant has one or more amino acid deletions, substitutions or additions and is capable of binding an anti-Rep antibody specific for a Rep protein having the amino acid sequence of SEQ ID NO: 1 or SEQ ID NO: 8.

3. The in-vitro method of claim 2, wherein the anti-Rep antibodies bind to an epitope that is within an amino acid sequence selected from the group consisting of amino acids from 1 to 136, from 137 to 229 and from 230 to 324 of SEQ ID NO: 1.

4. The in-vitro method of claim 2 or 3, wherein the antibody specific for Rep protein binds to an epitope comprised by SEQ ID NO: 2 or SEQ ID NO: 3.

5. The in-vitro method of any of claims 2 to 4, wherein the sample from a subject is selected from the group consisting of a cancerous prostate tissue, peripheral tissue surrounding the cancerous tissue, (benign) hyperplasia.

6. The in-vitro method of any of claims 2 to 5, wherein additionally CD68 positive cells are detected in the sample by an anti-CD68 antibody.

## Patentansprüche

1. In-vitro-Verwendung des Rep-Proteins der Gruppe 1 von Fleisch- und Milchfaktoren von Rindern ("Bovine Meat and Milk Factor Group 1"; BMMF1) als Biomarker für Prostatakrebs, wobei das Rep-Protein ein MSBI1-Genom-kodiertes Rep-Protein (MSBI1 Rep), kodiert durch MSBI1.176, umfassend die in SEQ ID NO: 1 dargestellte Aminosäuresequenz, ein MSBI2-Genom-kodiertes Rep-Protein (MSBI2 Rep), kodiert durch MSBI2.176, umfassend die in SEQ ID NO: 8 dargestellte Aminosäuresequenz, ein CMI1-Genom-kodiertes Rep-Protein (CMI1 Rep), kodiert durch CMI1.252, umfassend die in SEQ ID NO: 10 dargestellte Aminosäuresequenz, ein CMI2-Genom-kodiertes Rep-Protein (CMI2 Rep), kodiert durch CMI2.214, umfassend die in SEQ ID NO: 11 dargestellte Aminosäuresequenz oder ein CMI3-Genom-kodiertes Rep-Protein (CMI3 Rep), kodiert durch CMI3.168, umfassend die in SEQ ID NO: 12 dargestellte Aminosäuresequenz, oder eine Variante des Proteins mit SEQ ID NO: 1 oder SEQ ID NO: 8 mit einer Homologie von mindestens 90% ist, wobei die Variante eine oder mehrere Aminosäuredeletionen, -substitutionen oder - additionen aufweist und in der Lage ist, einen Anti-Rep-Antikörper, der für ein Rep-Protein mit der Aminosäuresequenz SEQ ID NO: 1 oder SEQ ID NO: 8 spezifisch ist, zu binden.

2. In-vitro-Verfahren zum Bereitstellen einer Diagnose oder Prädisposition für Prostatakrebs bei einem Patienten, das den Schritt des Nachweises von Rep-Protein in einer Probe eines Patienten durch Anti-Rep-Antikörper umfasst, wobei die Anti-Rep-Antikörper für MSBI1 Rep, MSBI2 Rep, CMI1 Rep, CMI2 Rep und/oder CMI3 Rep gemäß Anspruch 1 oder für die Variante des Proteins mit SEQ ID NO: 1 oder SEQ ID NO: 8 mit einer Homologie von mindestens 90% spezifisch sind, wobei die Variante eine oder mehrere Aminosäuredeletionen, -substitutionen oder -additionen aufweist und in der Lage ist, einen Anti-Rep-Antikörper, der für ein Rep-Protein mit der Aminosäuresequenz SEQ ID NO: 1 oder SEQ ID NO: 8 spezifisch ist, zu binden.

3. In-vitro-Verfahren nach Anspruch 2, wobei die Anti-Rep-Antikörper an ein Epitop binden, das sich innerhalb einer Aminosäuresequenz befindet, die aus der Gruppe, die aus den Aminosäuren 1 bis 136, 137 bis 229 und 230 bis 324 von SEQ ID NO: 1 besteht, ausgewählt ist.

4. In-vitro-Verfahren nach Anspruch 2 oder 3, wobei der für das Rep-Protein spezifische Antikörper an ein Epitop, das von SEQ ID NO: 2 oder SEQ ID NO: 3 umfasst ist, bindet.

5. In-vitro-Verfahren nach einem der Ansprüche 2 bis 4, wobei die Probe aus einem Patienten aus der Gruppe bestehend aus krebsartigem Prostatagewebe, peripherem Gewebe, das das krebsartige Gewebe umgibt oder (benigner) Hyperplasie ausgewählt ist.

6. In-vitro-Verfahren nach einem der Ansprüche 2 bis 5, wobei zusätzlich CD68-positive Zellen in der Probe durch einen Anti-CD68-Antikörper nachgewiesen werden.

## Revendications

1. Utilisation *in vitro* d'une protéine Rep des facteurs de la viande et du lait bovins de groupe 1 (BMMF1) en tant que biomarqueur pour le cancer de la prostate, dans laquelle la protéine Rep est une protéine Rep codée dans le génome MSBI1 (MSBI1 Rep) qui est codée par MSBI1.176 comprenant la séquence d'acides aminés telle que représentée dans SEQ ID NO: 1, une protéine Rep codée dans le génome MSBI2 (MSBI2 Rep) qui est codée par MSBI2.176 comprenant la séquence d'acides aminés telle que représentée dans SEQ ID NO: 8, une protéine Rep codée dans le génome CMI1 (CMI1 Rep) qui est codée par CMI1.252 comprenant la séquence d'acides aminés telle que représentée dans SEQ ID NO: 10, une protéine Rep codée dans le génome CMI2 (CMI2 Rep) qui est codée par CMI2.214 comprenant la séquence d'acides aminés telle que représentée dans SEQ ID NO: 11, une protéine Rep codée dans le génome CMI3 (CMI3 Rep) qui est codée par CMI3.168 comprenant la séquence d'acides aminés telle que représentée dans SEQ ID NO: 12, ou un variant de la protéine de SEQ ID NO: 1 ou SEQ ID NO: 8 présentant une homologie d'au moins 90 %, dans laquelle le variant comporte une ou plusieurs délétions, substitutions ou additions d'acides aminés et est capable de se lier à un anticorps anti-Rep spécifique pour une protéine Rep possédant la séquence d'acides aminés de SEQ ID NO: 1 ou SEQ ID NO: 8.

2. Procédé *in vitro* pour établir un diagnostic ou une prédisposition pour le cancer de la prostate chez un sujet comprenant l'étape consistant à détecter une protéine Rep dans un échantillon d'un sujet par des anticorps anti-Rep, dans lequel les anticorps anti-Rep sont spécifiques pour MSBI1 Rep, MSBI2 Rep, CMI1 Rep, CMI2 Rep et/ou CMI3 Rep selon la revendication 1, ou pour le variant de la protéine de SEQ ID NO: 1 ou SEQ ID NO: 8 présentant une homologie d'au moins 90 %, dans lequel le variant comporte une ou plusieurs délétions, substitutions ou additions d'acides aminés et est capable de se lier à un anticorps anti-Rep spécifique pour une protéine Rep possédant la séquence d'acides aminés de SEQ ID NO: 1 ou SEQ ID NO: 8.

3. Procédé *in vitro* selon la revendication 2, dans lequel les anticorps anti-Rep se lient à un épitope qui est compris au sein d'une séquence d'acides aminés sélectionnée dans le groupe consistant en les acides aminés 1 à 136, 137 à 229 et 230 à 324 de SEQ ID NO: 1.

4. Procédé *in vitro* selon la revendication 2 ou 3, dans lequel l'anticorps spécifique pour une protéine Rep se lie à un épitope compris par SEQ ID NO: 2 ou SEQ ID NO: 3.

5. Procédé *in vitro* selon l'une quelconque des revendications 2 à 4, dans lequel l'échantillon d'un sujet est sélectionné dans le groupe consistant en un tissu prostatique cancéreux, un tissu périphérique entourant le tissu cancéreux, une hyperplasie (bénigne).

6. Procédé *in vitro* selon l'une quelconque des revendications 2 à 5, dans lequel en outre des cellules positives pour CD68 sont détectées dans l'échantillon par un anticorps anti-CD68.
